(19) Europäisches Patentamt
European Patent Office
Office européen des brevets

(11) Veröffentlichungsnummer: **0 004 052**
**B1**

(12) # EUROPÄISCHE PATENTSCHRIFT

(45) Veröffentlichungstag der Patentschrift: **06.10.82**

(51) Int. Cl.³: **C 07 D 307/93**, C 11 B 9/00 // C07C69/74, C07C35/02

(21) Anmeldenummer: **79100645.5**

(22) Anmeldetag: **05.03.79**

(54) **13-Oxabicyclo(10.3.0)pentadecan, dessen Herstellung und Verwendung als Riechstoff, sowie dieses enthaltende Riechstoffkompositionen.**

(30) Priorität 09.03.78 DE 2810107

(43) Veröffentlichungstag der Anmeldung: **19.09.79 Patentblatt 79/19**

(45) Bekanntmachung des Hinweises auf die Patenterteilung: **06.10.82 Patentblatt 82/40**

(84) Benannte Vertragsstaaten: **BE CH FR GB NL**

(56) Entgegenhaltungen:
**DE-A-2 136 496**
**DE-B-2 209 372**
**BULLETIN OF THE CHEMICAL SOCIETY OF JAPAN; Band 46, Nr. 8, August 1973, Tokyo, A. UCHIDA et al.: »The Prins Reaction of Cyclo-octene and Cyclododecene« Seiten 2512–2525**

(73) Patentinhaber: **Henkel Kommanditgesellschaft auf Aktien, Postfach 1100 Henkelstrasse 67, D-4000 Düsseldorf 1 (DE)**

(72) Erfinder: **Meins, Peter, Dr.**, Schillerstrasse 13, **D-4020 Mettmann (DE)**
Erfinder: **Bruns, Klaus, Dr., Notburgaweg 6, D-4150 Krefeld-Traar (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

»13-Oxabicyclo[10.3.0]pentadecan, dessen Herstellung und Verwendung als Riechstoff sowie dieses enthaltende Riechstoffkompositionen«

Oxabicycloalkene sind bereits als Riechstoffe und als Riechstoffvorprodukte literaturbekannt. So werden z. B. in den deutschen Offenlegungsschriften 2 026 056 und 2 136 496 Verfahren zur Herstellung solcher Ringsysteme und insbesondere von 13-Oxabicyclo[10.3.0]pentadecen-(1) angegeben, es werden dort jedoch keine Angaben über die Art der Duftnoten der erhaltenen Verbindungen gemacht. In der deutschen Auslegeschrift 2 209 372 wird 3-Oxabicyclo[10.3.0]pentadecen-(6) als Riechstoff mit stark holziger Moschus-Ambra-Duftnote beschrieben. Demgegenüber wurde gefunden, daß 13-Oxabicyclo[10.3.0]pentadecan einen neuen Riechstoff mit warmer Ambranote von außergewöhnlicher Qualität bezüglich Haftfestigkeit und Kompositionsfähigkeit darstellt.

Die Herstellung der erfindungsgemäßen neuen Verbindung erfolgt nach an sich bekannten Syntheseverfahren der organischen Chemie. Als Ausgangsmaterial für die Synthese dient Cyclododecanon (I), das mit Bromessigester nach Reformatsky zum entsprechenden Hydroxyester (II) umgesetzt wird, der sich im stark sauren Milieu unter Erhitzen leicht in das Lacton (III) umlagert. Anschließend wird das Lacton mit Natriumborhydrid in Isopropanol in das Diol (IV) übergeführt, aus dem durch Dehydratisierung in Toluol in Gegenwart von p-Toluolsulfonsäure das 13-Oxabicyclo[10.3.0]pentadecan (V) als Gemisch zweier Stereoisomerer etwa im Verhältnis 2 : 1 erhalten wird. Die Herstellung verläuft gemäß nachstehendem Reaktionsschema:

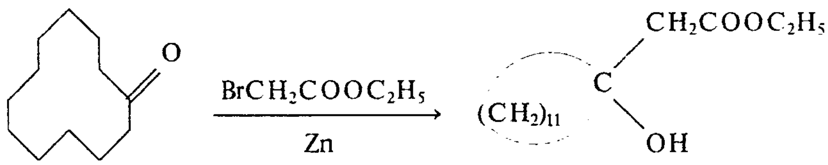


(I) (II)

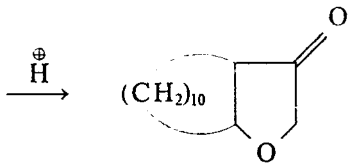

(III)

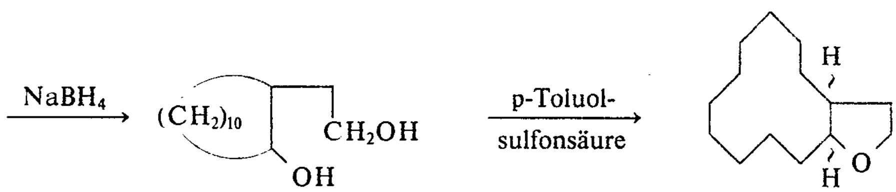


(IV) (V)

Das 13-Oxabicyclo[10.3.0]pentadecan zeichnet sich durch eine angenehme, warme Ambra-Note sowie eine außergewöhnliche Haftfestigkeit aus. Ein weiterer Vorteil ist seine sehr gute Kombinationsfähigkeit zu neuartigen Kompositionen, denen es gleichfalls eine besondere Haftfestigkeit verleiht.

Das 13-Oxabicyclo[10.3.0]pentadecan kann mit anderen Riechstoffen in den verschiedensten Mengenverhältnissen zu neuen Riechstoffkompositionen gemischt werden. Im allgemeinen bewegt sich sein Anteil in den Riechstoffkompositionen in den Mengen von 1 bis 50 Gewichtsprozent, bezogen auf die gesamte Komposition. Derartige Kompositionen können direkt als Parfüm oder auch zur Parfümierung von Kosmetika wie Cremes, Lotionen, Duftwässern, Aerosolen, Toiletteseifen dienen. Sie können aber auch zur Geruchsverbesserung technischer Produkte wie Wasch- und Reinigungsmitteln, Weichspülern, Textilbehandlungsmitteln eingesetzt werden. Zur Parfümierung der verschiedenen Produkte werden diesen die Kompositionen im allgemeinen in Konzentrationen von 0,05 bis 2 Gewichtsprozent zugesetzt.

Die nachfolgenden Beispiele sollen den Gegenstand der Erfindung näher erläutern.

## Beispiele

### 1. Herstellung des 13-Oxabicyclo[10.3.0]pentadecans.

a) Herstellung des Hydroxyesters

In eine Suspension von 33 g Zinkpulver in Toluol/Benzol (140/160 ml), das mit einigen Kristallen Jod aktiviert wurde, wurde unter Rühren und Rückfluß während 4 Stunden eine Lösung von 182 g (1 Mol) Cyclododecanon und 167 g (1 Mol) Bromessigsäureethylester in Toluol/Benzol (140/160 ml) zugetropft und danach weitere 2 Stunden unter Rückfluß erhitzt. Das verwendete Zinkpulver wurde in folgender Weise vorbehandelt: Zinkpulver wurde ca. 20 Minuten in 10%iger Salzsäure gerührt, abgesaugt, mit Wasser neutral gewaschen und mit Aceton trocken gewaschen. Anschließend wurde bei 50° C im Vakuum getrocknet.
Nach dem Abkühlen des Reaktionsgemisches wurde nicht umgesetztes Zinkpulver unter Rühren und Kühlung mit ca. 300 ml eiskalter Schwefelsäure (10%ig) gelöst, die organische Phase abgetrennt, mit 2n-Sodalösung, 2n-Schwefelsäure und schließlich mit Wasser neutral gewaschen. Nach dem Trocknen über Natriumsulfat wurde das Lösungsmittel entfernt und der erhaltene Hydroxyester im Ölpumpenvakuum vom nicht umgesetzten Cyclododecanon befreit.

b) Herstellung des Lactons

Das erhaltene Hydroxyester-Rohprodukt (270 g) wurde unter kräftigem Rühren bei 60° C zu 1080 ml 80%iger Schwefelsäure gegeben. Das Gemisch wurde 1 Stunde bei 60° C intensiv gerührt und nach Abkühlen auf Eis gegossen und so lange nachgerührt, bis das Eis vollständig geschmolzen war. Danach wurde mit Äther extrahiert, die ätherische Phase mit 2n-Sodalösung und anschließend mit Wasser neutral gewaschen. Nach Trocknen über Natriumsulfat und Abdestillieren des Lösungsmittels hinterblieb das Lacton als kristalline Masse.

c) Herstellung des Diols

Das erhaltene rohe Lacton (204 g) wurde in 3600 ml Isopropanol gelöst. Unter Rühren wurden 45,6 g (1,2 Mol) Natriumborhydrid zugesetzt und das Gemisch 3 Stunden unter Rühren am Rückfluß erhitzt. Nach beendeter Reaktion wurde das Gemisch in die zweifache Menge Wasser eingegossen, das Gemisch mehrfach mit Äther extrahiert, die Extrakte getrocknet, das Lösungsmittel entfernt und das Rohprodukt im Ölpumpenvakuum destilliert.
Das erhaltene Diol stellt eine farblose viskose Flüssigkeit dar mit einem $Kp_{0,6}$ von 172—175° C.

d) Überführung des Diols in das 13-Oxabicyclo[10.3.0]pentadecan

Das erhaltene Diol (208 g) wurde in 640 ml Toluol gelöst, 17,2 g p-Toluolsulfonsäure zugesetzt und die Lösung 2 Stunden am Wasserabscheider erhitzt. Nach Beendigung der Reaktion wurde mit 2n-Sodalösung, danach mit Wasser neutral gewaschen und die Lösung über Natriumsulfat getrocknet. Nach Entfernen des Lösungsmittels wurde im Ölpumpenvakuum destilliert. Das erhaltene 13-Oxabicyclo[10.3.0]pentadecan ist eine farblose Flüssigkeit mit folgenden Kennzahlen:

$Kp_{0,15}$ 92—94° C
IR (Film): 1000—1100, max. 1057/cm ($CH-O-CH_2$)
1H-NMR: $\delta$ 3,82 (t, 2H; $O-CH_2$)

Der Geruch wird als eine warme Ambra-Note charakterisiert.

### 2. Kompositionen

a) Riechstoffkomposition Holz-Ambra-Base

| | |
|---|---|
| 13-Oxabicyclo[10.3.0]pentadecan | 250 Gew.-Teile |
| Boisambrene® (Henkel KGaA) | 250 Gew.-Teile |
| Cedrylacetat | 250 Gew.-Teile |
| Zimtalkohol | 80 Gew.-Teile |
| Jonon | 70 Gew.-Teile |
| Galaxolid® (IFF) | 70 Gew.-Teile |
| Methylnonylacetaldehyd 5%ig in Diethylphthalat | 30 Gew.-Teile |

b) Künstliche Ambra-Komposition

| | |
|---|---|
| 13-Oxabicyclo[10.3.0]pentadecan | 250 Gew.-Teile |
| Boisambrene® (Henkel KGaA) | 250 Gew.-Teile |
| Muskatellersalbeiöl | 140 Gew.-Teile |
| Methyljonon | 100 Gew.-Teile |
| Cedrenol | 50 Gew.-Teile |
| 2-Acetyl-4-isopropyl-5,5-dimethyl-1,3-dioxan | 50 Gew.-Teile |
| Ketonmoschus | 50 Gew.-Teile |
| Res. Benzoe Siam | 30 Gew.-Teile |
| Res. Tolu | 30 Gew.-Teile |
| Pentadecanolid | 20 Gew.-Teile |
| Patchouliöl | 20 Gew.-Teile |
| Ethylvanilin | 10 Gew.-Teile |

**Patentansprüche**

1. 13-Oxabicyclo[10.3.0]pentadecan.
2. Verfahren zur Herstellung von 13-Oxabicyclo[10.3.0]pentadecan durch Umsetzen von Cyclododecanon mit Bromessigester zum entsprechenden Hydroxyester, dessen Umlagerung zum Lacton, Überführen des Lactons mittels Natriumborhydrid zum Diol und Dehydratisierung des Diols zu dem 13-Oxabicyclo[10.3.0]pentadecan in Form des Gemisches zweier Stereoisomerer.
3. Verwendung von 13-Oxabicyclo[10.3.0]pentadecan als Riechstoff.
4. Riechstoffkompositionen, dadurch gekennzeichnet, daß sie 13-Oxabicyclo[10.3.0]pentadecan in einer Menge von 1—50 Gewichtsprozent, bezogen auf die gesamte Komposition, enthalten.

**Claims**

1. 13-oxabicyclo-[10.3.0]-pentadecane.
2. A process for the production of 13-oxabicyclo-[10.3.0]-pentadecane by reacting cyclododecanone with bromoacetic ester to form the corresponding hydroxy ester, rearranging the hydroxy ester to form the lactone, converting the lactone into the diol using sodium borohydride and dehydrating the diol to form 13-oxabicyclo-[10.3.0]-pentadecane in the form of a mixture of two stereo-isomers.
3. The use of 13-oxabicyclo-[10.3.0]-pentadecane as a perfume.
4. A perfume composition, characterised in that it contains 13-oxabicyclo-[10.3.0]-pentadecane in a quantity of from 1 to 50% by weight, based on the composition as a whole.

**Revendications**

1. Le 13-oxabicyclo[10.3.0]pentadécane.
2. Procédé de préparation du 13-oxabicyclo[10.3.0]pentadécane par réaction de la cyclododécanone avec le bromacétate d'éthyle conduisant à l'hydroxyester correspondant, transposition de ce dernier en lactone, conversion de la lactone à l'aide du borohydrure de sodium en diol et déshydratation de ce dernier en le 13-oxabicyclo[10.3.0]-pentadécane à l'état de mélange des deux stéréoisomères.
3. Utilisation du 13-oxabicyclo[10.3.0]pentadécane en tant que matière aromatique.
4. Compositions de parfums, caractérisées en ce qu'elles contiennent le 13-oxabicyclo[10.3.0]pentadécane en quantités de 1 à 50% en poids de la composition totale.